(19) Europäisches Patentamt

European Patent Office

Office européen des brevets

(11) Publication number: **0 362 758 B1**

(12) **EUROPEAN PATENT SPECIFICATION**

(45) Date of publication of patent specification: **13.04.94** (51) Int. Cl.⁵: **C12N 5/00, A61K 35/18**

(21) Application number: **89118215.6**

(22) Date of filing: **02.10.89**

(54) Electro-insertion of proteins into animal cell membranes.

(30) Priority: **05.10.88 US 254550**
**19.09.89 US 407664**

(43) Date of publication of application:
**11.04.90 Bulletin 90/15**

(45) Publication of the grant of the patent:
**13.04.94 Bulletin 94/15**

(84) Designated Contracting States:
**AT BE CH DE ES FR GB GR IT LI LU NL SE**

(56) References cited:
**EP-A- 0 101 341**
**EP-A- 0 298 280**
**FR-A- 2 374 045**

**BIOCHEMICAL AND BIOPHYSICAL RE-SEARCH COMMUNICATIONS, vol. 159, no. 1, 28thFebruary 1989, pages 34-40, Academic Press, Inc.; Y. MOUNEIMNE et al.:"Electro-insertion of xeno-glycophorin into the red blood cell membrane"**

(73) Proprietor: **TEXAS A&M UNIVERSITY SYSTEM**

**College Station, TX 77843(US)**

(72) Inventor: **Mouneimne, Youssef**
**505 Boyett, Nr.4**
**College Station, TX 77840(US)**
Inventor: **Tosi, Pierre-François**
**1300 Briarcliff, Nr.131**
**Bryan, TX 77802(US)**
Inventor: **Nicolau, Yves-Claude**
**2100 Farley**
**College Station, TX 77840(US)**

(74) Representative: **Cohausz & Florack Patentan-wälte**
**Postfach 33 02 29**
**D-40435 Düsseldorf (DE)**

EP 0 362 758 B1

CHEMICAL ABSTRACTS, vol. 110, no. 7, 13th February 1987, page 356, abstract no.54113x, Columbus, Ohio, US; J.G. BLISS et al.: "Electroporation: the population distribution of macromolecular uptake and shape changes in red blood cells following a single 50 mus square wave pulse", & BIOLECTROCHEM. BIORNERG. 1988, 20(1-3), 57-718, 20(1-3), 57-71

CHEMICAL ABSTRACTS, vol. 107, no. 7, 17th August 1987, page 30, abstract no.51519m, Columbus, Ohio, US; M.M, JASTREBOFF et al.: "Use of electroporation tostudy the cytotoxic effects of fluorodeoxyuridylate in intact cells",& BIOCHEM. PHARMACOL. 1987, 36(8), 1345-8

BIOLICAL ABSTRACTS, vol. 80, no. 1, 1985, abstract no. 622, Biological Abstracts, Inc.,Philadelphia, PA, US; E.H. SERPERSU et al.: "Reversible and irreversible modification of erytrocyte membrane permeability by electric field", & BIOCHIM. BIOPHYS ACTA 812(3): 779-795. 1985

## Description

## BACKGROUND OF THE INVENTION

### Field of the Invention

The present invention concerns a method for incorporating a protein into an animal cell membrane by exposing the cell to electric field pulses.

### Background Information

Electroporation or electrofusion is described by Zimmerman, Neumann, Tsong, Kinosita, Tessie, Deutike, etc., Cell Fusion, ed., A.E. Sowers, Plenum Press, New York 1987; Electro Genetic-Physical and Biological Aspect of Cellular Electromanipulation, Gunter A. Hofman and Glen A. Evans, IEEE Eng. in Med. and Biol. Magazine, Dec. 1986, p.6, "Dielectric Breakdown - Fusion/Electroporation".

The following papers described spontaneous insertion of a variety of proteins into cell membranes: Medof et al, "Inhibition of Complement Activation of the Surface of Cells After Incorporation of Decay-Accelerating Factor (DAF) Into their Membranes", J. Exp. Med., 160, 1558-1578 (1984); Zalman et al, "Deficiency of the Homologous Restriction Factor in Paroxysmal Nocturnal Hemoglobinuria", Journal of Exp. Med., 165, 572-577 (1987); "Incorporation and Assymmetric Orientation of Glycophorin in Reconstituted Protein-Containing Vesicles", Eur. J. Biochem., 86, 539-546 (1978); Zalman et al, "Inhibition of Antibody-Dependent Lymphocyte Cytotoxicity by Homologous Restriction Factor Incorporated Into Target Cell Membranes", J. Exp. Med., 166, 947-955 (1987); Scotto et al, "Reconstitution of Membrane Proteins", The Journal of Biological Chemistry, 263, 18500-18506, (1988). These papers, however, do not concern electro-insertion.

## SUMMARY OF THE INVENTION

The present invention concerns a method of incorporating a protein into a cell membrane (e.g., an animal cell membrane), comprising exposing a cell to an electric field while the cell is suspended in an electro-insertion medium in the presence of a buffered solution (suspension) of the protein(s) to be inserted, the protein bearing a hydrophobic membrane spanning sequence and then contacting the resultant cell with a resealing medium.

## BRIEF DESCRIPTION OF THE DRAWINGS

Fig. 1a is a photograph depicting aggregation of Murine red blood cells bearing human glycoporin shown by fluorescence microscopy.

Fig. 1b is another photograph depicting aggregation of murine red blood cells bearing human glycophorin shown by fluorescence microscopy.

Figs. 2a and 2b are fluorescence histograms of cell number versus fluorescence intensity obtained by cytofluorometry (EPICS PROFILE). The control sample without electric pulse (Fig. 2a) does not show any high fluorescent cells on 1213 cells counted, while the pulsed sample (Fig. 2b) shows that 10.4% of 1742 Murine red blood cells contain about $2.5 \times 10^5$ FITC corresponding to glycophorin insertion.

Figs. 3a and 3b are photographs depicting agglutination of human red blood cells bearing biotynilated glycophorin gas shown by white light microscopy (Fig. 3a) and fluorescence microscopy (Fig. 3b).

Figs. 4a and 4b are photographs of rabbit red blood cells bearing electro-inserted human glycophorin after reaction, with 10F7 anti-human glycophorin antibody and a secondary fluorescent antibody (Goat anti-mouse phycoerythrin) as it appears with fluorescence (Fig. 4a) and direct light (Fig. 4b).

Figs. 5a and 5b are photographs of human red blood cells bearing electro-inserted CD4 after reaction with OKT4C and Leu3a monoclonal antibodies and goat anti-mouse phycoerythrin antibody. Notice the bright fluorescent points (Fig. 5a) characteristic of the capping of the inserted proteins upon reaction with monoclonal antibodies. Under direct light (Fig. 5b), the cells appear not to be affected by the treatment.

Fig. 6 is a plot which depicts the flow cytometry analysis of rabbit red blood cells-glycophorin (---), which shows a fluorescence intensity shift with repsect to the control cells (—). The intensity level corresponds to 4,000 glycophorin/cell.

Fig. 7 is a plot which depicts the flow cytometry analysis of rabbit red blood cells CD4 (---), which shows a fluorescence intensity shift with respect to the control cells (—). The intensity level corresponds to 2,000 CD4 molecules/cell.

Fig. 8 is a series of bar graphs which shows that the amount of inserted protein molecules per cell (represented as relative fluorescence intensity), detected by flow cytometry, depends on the number of applied electrical pulses. The system employed was murine RBC-glycophorin.

Fig. 9 is a plot showing that the insertion efficiency (I = inserted protein moleucles per cell/added protein molecules per cell) increases with [the] hematocrit. The system used was murine red blood cell-glycophorin.

Fig. 10 is a plot depicting that the number of inserted protein molecules per cell depends on the concentration of proteins. The system utilized was murine red blood cells-glycophorin.

Fig. 11 is a graph showing that the insertion efficiency of PCNBD (represented as relative fluorescence intensity) depends on the hematocrit. The system used was murine red blood cells.

Fig. 12 is a plot showing the life span of rabbit red blood cells ($^{51}$Cr labelled) subjected to the insertion of $^{125}$I-CD4 and $^{125}$I-glycophorin.

## DETAILED DESCRIPTION OF THE INVENTION

The present invention concerns the incorporation of a protein bearing a hydrophobic membrane spanning sequence through a cell membrane by exposing the cell to an electric field, preferably one or more electric field pulses, while the cell is suspended in an electro-insertion medium and thereafter contacting the resultant cell with a resealing medium.

It is preferred to carry out the inventive method at 2°C to 39°C, with 37°C being particularly preferred.

It is preferred to carry out the inventive method up to and including resealing in 1 to 4 hours, with 3 hours being particularly preferred.

Cells suspended in a liquid can be modeled as a structure consisting of a non-conducting membrane with aqueous solutions on both sides. Exposure to an electric field leads to charge separation in the membrane similar to the charge separation in the dielectric layers of an electrical capacitor. This results in a transmembrane potential difference. Opposite electrical charges on the membrane attract each other, exerting a pressure on the membrane which can induce membrane thinning. At a critical potential difference (field strength), localized breakdown of the membrane occurs and pores are formed, allowing a flow of medium or cytoplasm. Removal of the field can lead to healing (closing) of the pores, provided that the field strength and pulse width are not excessive.

The present invention is applicable to cells generally because it deals with the lipid membrane of cells, taking into account a possible need for special conditions for certain types of cells. Non-limiting examples of cells for use in the present invention include red blood cells, lymphocytes, monocytes, mammalian culture cells of all types, fibroblasts, hepatocytes, endothelial cells, neurons and macrophages, just to mention a few. The invention is also applicable for plant cells and microorganisms, e.g., bacteria and yeast.

Any cell can be subjected to electro-insertion according to the invention with, if needed, special electrical, chemical and/or biological conditions, i.e., human and monkey cell lines (Hela, CVI, Vero), mouse and hamster cell lines (N1H3T3, CHO), insect cells, yeast, bacteria and plant cells, just to name a few.

The cell is preferably an animal cell, and most preferably a warm blooded animal. The cell can be from a human or another warm blooded animal, e.g., cow, cat, rabbit, monkey, mouse, goat, sheep or horse. Preferably the cell is found in the bloodstream. More preferably the cell is an erythrocyte. The cell could also be a human lymphoma T cell or culture cells, e.g., nucleated animal cells in culture, for example, CEM cells.

Any protein bearing a hydrophobic membrane spanning sequence can be used in the invention. Besides glycophorin and CD4, CD2, CD3, MHC Class I and II, LDL receptor, insulin receptor, different hormone receptors or any genetically engineered soluble protein bearing a specific transmembrane hydrophobic sequence can be employed. The preferred proteins for insertion are human glycophorin and human CD4.

The electro-insertion occurs according to the empiric law

$$V = V_o + 1.5 \, R \, E \cos (\theta),$$

where V is the resultant membrane potential and $V_o$ is the natural membrane potential. R is the cell radius, E is the field strength, and 0 is the angle between a given membrane site and the field direction.

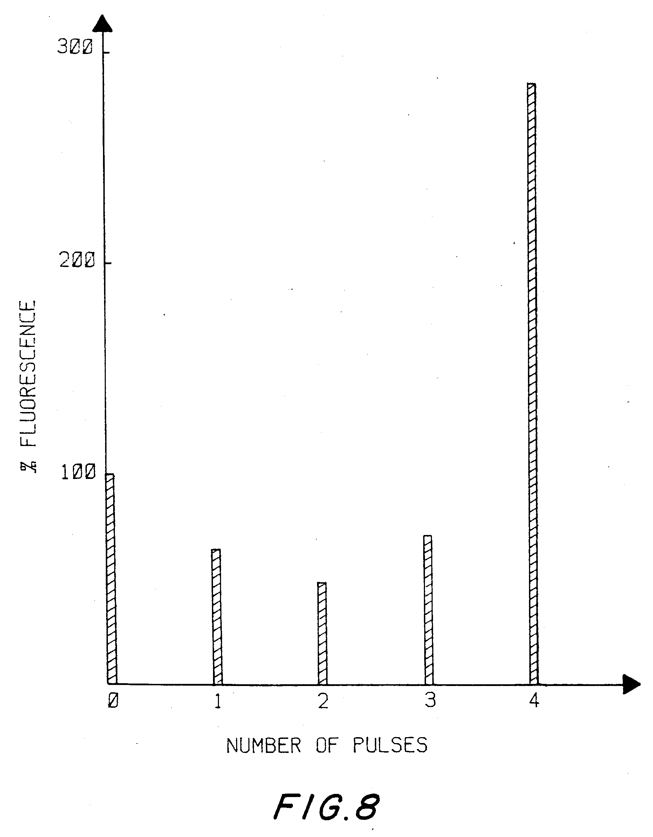

Reduction of the membrane potential by pretreating the red blood-cells with ionophor A-23187 leads to a 85% decrease of insertion efficiency of glycophorin into rabbit red blood cells. Obviously, with no external field, minimum inserted molecules per cell are obtained. Application of four electrical pulses produces a high insertion. Fig. 8 shows the fluorescence intensity relative to the insertion of human glycophorin into murine red blood cells, with no external field and using up to four electrical pulses.

The maximum number of pulses for use in the invention is limited only by the behavior of the cell under the multiple pulses and the time limiting biological and chemical conditions of the procedure. The maximum could be 100 pulses, but there is no need to employ such number of pulses as long as a maximum efficiency for a limited number of pulses is achieved. The aim of the use of multiple pulses is to increase the probability of the exposure of cells to the electric field.

In order to achieve intact cells any pulse height equal to or below the critical field for electroporation can be used, but the insertion decreases with decreased height value. Pulses equal to or higher than the critical field are also efficient, but the cells swell or lyse. The same occurs for the duration, lower pulses (i.e. < 400 µs), but they are also good but less efficient. Longer pulses may be more efficient, but they cause heat increase and damage, and depending on the electrical field intensity, up to second(s) pulses can be used. In general, the applied field and the duration of the pulse are inversely proportional and cell type dependent. The interval between pulses can be microseconds to minutes; the only limitation is the heat dissipation of the electric chamber and the recovery of the cells after each pulse.

The pulse shape need not be square, and any pulse shape is efficient as long as the required energy (E,T) is provided. An exponential decay, sawtooth, or even alternating pulses could be used. The electrical field is not necessarily pulsed and could be also continuous (dc).

One set of preferred characteristics of the electric field pulses for use according to the invention is 8 pulses applied sequentially with a time interval between each pulse of five seconds. Preferably the electric field pulse has a height of up to 1.3 kv/cm for human red blood cells, 1.6 kv/cm for rabbit red blood cells and 1.5 to 2.1 kv/cm for murine red blood cells. Preferably, in one embodiment, the pulse duration is at least 400 $\mu$ seconds long. The pulse duration in another preferred embodiment is lms with a set of four pulses applied sequentially with a time interval between each pulse of 15 minutes. Preferably the pulses are square pulses. Preferably the electric field pulse is no higher than the critical electric field for electroporation of the cell.

Any isotonic medium can be used as the electro-insertion medium employed in the invention, depending on the cells and the pulse generator power. In one embodiment of the invention, besides mannitol and histidine, NaCl, KCl, $Na_2HPO_4$, $NaH_2PO_4$, some carbohydrates like stachyose, inuline to any tetrasaccharide can be utilized. Physiological pH is preferred, but any pH can be used depending on the use, cells and proteins.

The electro-insertion medium for use in the present invention preferably contains in one embodiment a mixture of mannitol and histidine, preferably at a pH of 7.8. In another preferred embodiment, PBS at a pH of 8.8 is utilized.

The resealing medium for use in the present invention preferably is an aqueous medium containing one or more of KCl, NaCl, BSA, HSA, $MgCl_2$ or glucose in PBS, preferably at a pH of 7.4. $MgCl_2$ can also be used. Physiological pH is preferred for red blood cells (pH = 7.4).

In a preferred embodiment of the invention, after resealing, [an] incubation with a preservation medium, e.g., PBS or "ADSOL", is carried out at 20 to 39°C, preferably 37°C for 1 to 2 hours, preferably 1 hour, and/or at 2 to 6°C, preferably 4°C, for 6 to 12 hours.

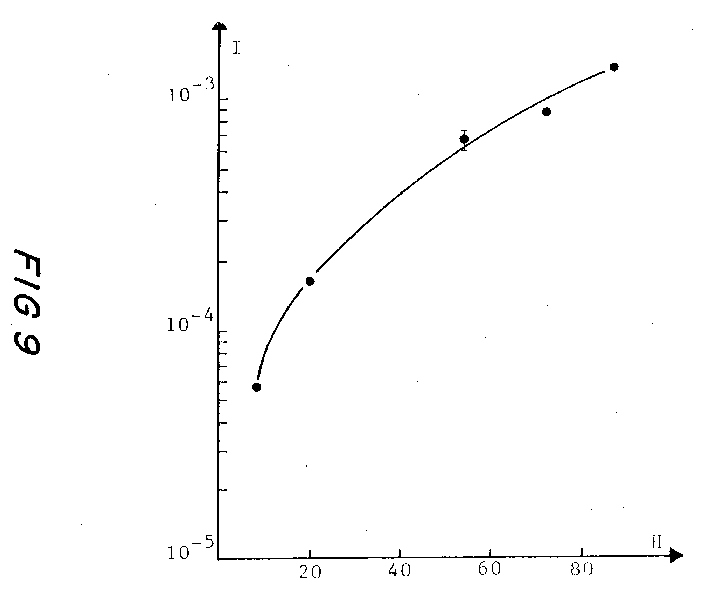

The electro-insertion depends on the suspension hematocrit. Fig. 9 shows that for a constant amount of added protein molecules per red blood cell, the insertion efficiency increases with the hematocrit. Preferably the hematocrit of the solution is 0.001% to 98%. Hematocrit can be defined as the fraction of total volume occupied by red blood cells.

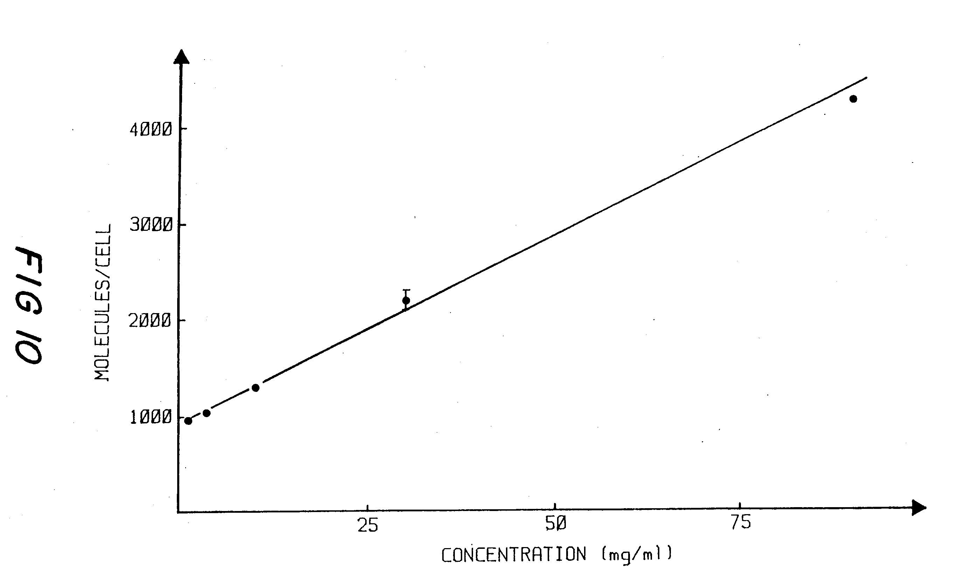

The amount of inserted protein molecules per cell increases with the added protein concentration. Any protein concentration can be used, including powder. A preferred concentration range is 0.01 mg/ml to 200 mg/ml, with 134 mg/ml being particularly preferred. Fig. 10 shows the relationship between the inserted glycophorin molecule per mouse red blood cell and the concentration of the added glycophorin at 86 hematocrit.

The hydrophobic interaction between the red blood cells' membrane and the inserted protein hydrophobic sequence is a key stone of electro-insertion. The dependence of the insertion efficiency on the suspension hematocrit may be considered as a dependence on the increase of the hydrophobic phase. Moreover, the electro-insertion increases the rate of natural transfer of phosphatidyl choline nitro-beazo 7-oxa-1,3-diazole (Acyl labeled-(PCNBD) (Avanti Polar Lipids Inc., Pelham, AL, U.S.A.) into the red blood cell membrane.

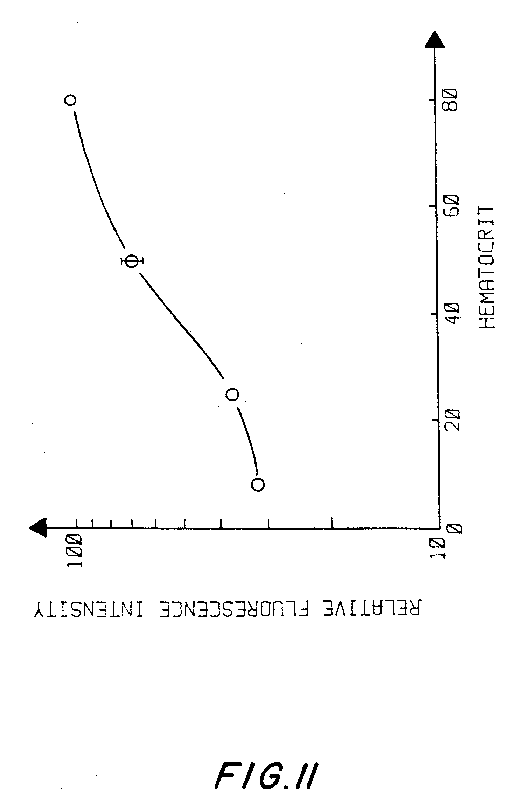

Fig. 11 shows the insertion efficiency as a function of hematocrit of murine red blood cells for the same amount of PCNBD. It was noticed that glycophorin (see Fig. 9) and PCNBD have similar behaviors, except that PCNBD shows higher values of insertion for lower hematocrit due the natural transfer. This similarity is further proof of the hydrophobic aspect of electro-insertion.

Moreover, it was noticed that adding L-alpha phosphatidic acid dipalmitoyl (Sigma, St. Louis, MO, U.S.A.) improves the insertion by 35%.

Finally, proteins without hydrophobic sequence such as human $\beta_2$ microglobulin (Sigma, St. Louis, MO, U.S.A.) were not inserted by the inventive method when detected by rabbit anti-human $\beta_2$ microglobulin antibody (Accurate Chemical and Scientific Corporation, Westburg, N.Y., U.S.A.).

A use for the present invention includes targeting of red blood cells as drug carriers, especially red blood cell-CD4$^+$ for Acquired Immunodeficiency Syndrome (AIDS). Red blood cells bearing different proteins or antigens or antibodies as targeting elements and all applications using cell surface receptors, in cancer and in infectious diseases, are also areas of use for the present invention. The product of the invention could be used as a vaccine, e.g., to treat malaria.

It is preferred that the ratio of protein molecules per cell be such that every cell is fully covered by the protein before the pulse. Depending on the state of the protein in solution (monomer or polymer), the ratio preferably is $10^6$ protein molecules per cell.

It is preferred to use a low concentration of calcium and magnesium ions, e.g., $\leq$ 0.5mM calcium and 0.5mM magnesium for red blood cells. For other than red blood cells, the concentrations depend on the cell, because very low concentrations (e.g., less than or equal to 0.5mM) give less adsorption of protein on the cell membrane and higher concentrations (for example, less than or equal to 0.5mM) lyse cells.

Magnesium and calcium help the protein to adsorb via electrostatic interaction on the membrane of the cell. Once this happens, the protein molecule is brought closer to the membrane than it was in solution. This adsorption is optimum at the given time, and it is preferred to apply electric pulses at this time.

The invention is now described with reference to the following non-limiting examples.

EXAMPLES

Example 1: Electro-insertion of Human Glycophorin in Murine and Rabbit Red Blood Cells

The pulse generator is a 606 Cober pulse generator. The "TEFLON" chamber used in the electro-insertion is cylindrical, 1.2 cm diameter, with each end formed by 1.2 cm x 2.5 cm stainless steel electrodes; the electrodes gap is 0.2 cm. Potential and current are monitored by a Nicolet 2090 digital oscilloscope.

Example 1a:

Red cells from freshly drawn murine blood are washed three times 0.145 NaCl in PBS 7.4 buffer, and two times in the electro-insertion medium (EM) (0.3 M mannitol and 6 mM histidine, pH = 7.8). After addition of 0.5 mM $CaCl_2$ and 0.5 mM $MgCl_2$, the cells are incubated 5 minutes on ice and then 5 minutes at 37°C (hematocrit 90%). Immediately after, a solution of 4 mg/ml of human glycophorin MM in 0.15 KCl and a given volume of the EM are added so that the protein concentration is 1.25 $\mu$g/$10^6$ cells. Then the suspension is incubated 5 minutes at 37°C. After this time, the suspension is centrifuged and the supernatant is removed, such that the remaining hematocrit is 16%. 25 $\mu$l of this final suspension is electropulsed at 4°C, with a pulse height of 1.5 kv/cm (below the critical field for electroporation of mouse red blood cells (2.6 kv/cm). Electro-insertion is accomplished by exposing the cell suspension (25 $\mu$l) to eight successive electric field square pulses with 5 second intervals. The pulse height depends on the cell size, but its value is below the critical value for electroporation.

The pulse length is 400 $\mu$s.

Then the cells are resealed for one hour at 37°C plus one more hour in the presence of the resealing medium (RM) (1.211 g KCl, 0.9g glucose, 0.5g BSA, 1.02g NaCl and phosphate buffered saline (PBS) 5mM pH 7.4 up to 250 ml) and finally washed one time with the RM and two times in 0.145 M NaCl PBS 7.4 buffer.

Example 1b:

Red cells from freshly drawn blood are washed five times in 0.14 NaCl in PBS 8.8 buffer. Membrane proteins lyophilized or suspended in 10PBS buffer pH 8.8 are added to the red blood cells pellet (hematocrit 90%). After 20 minutes incubation on ice and 15 minutes incubation at 37°C, four electrical pulses are applied at 37° with 15 minute intervals. Each pulse is 1 ms long and 1.3 kV/cm high for human, 1.6 KV/cm for rabbit or 2.1 kV/cm for murine red blood cells. The suspension is then incubated for two more hours at 37°C. Finally, red blood cells are washed one time with plasma and two times with 5 PBS pH 7.4. Another incubation at 50% hematocrit at 37°C in 5 PBS 7.4 or in preservation medium (the preservation medium "ADSOL" contains: 2.2g dextrose (hydrous) USP, 900 mg sodium chloride USP, 750 mg mannitol USP and 27 mg ADENINE in 100 ml (FENWAL Laboratories, Deerfield, IL, U.S.A.) may improve the life span of the inserted proteins into the red blood cell membranes.

Example 2:

CD4 receptor is inserted in the red blood cells' membrane (human, rabbit and murine) under the same conditions described in Example 1b reaching agglutinating densities when reacted with $gp_{120}$-expressing, HIV-infected H9 cells.

Example 3: Immunofluorescence Assay

After insertion of glycophorin into the red blood cell membrane, the washed cells are then incubated with 2 $\mu$l of 0.17 mg/ml 10F7 anti-glycophorin monoclonal antibody (Biomedical Sciences Division, Livermore National Laboratory, University of California) for 30 minutes at room temperature and then washed three times 0.145 M NaCl PBS 7.4 buffer. The cells are then incubated with phycoerythrin conjugated affinipure F(ab')2 fragment goat anti-mouse IgG (Jackson Immuno Research Laboratories, West Grove, Pa., U.S.A.) or fluoresceine conjugated affinipure F(ab')2 fragment rabbit anti-mouse IgG (Accurate Chemical and Scientific Corporation, Westburg, NY, U.S.A.) at room temperature for 30 minutes. The cells are finally washed three times in 0.145 M NaCl PBS 7.4 buffer.

Example 3a:

A control sample where the murine RBC (red blood cells) have undergone all the steps mentioned in Example 1a, but without electroporation, is used as a reference.

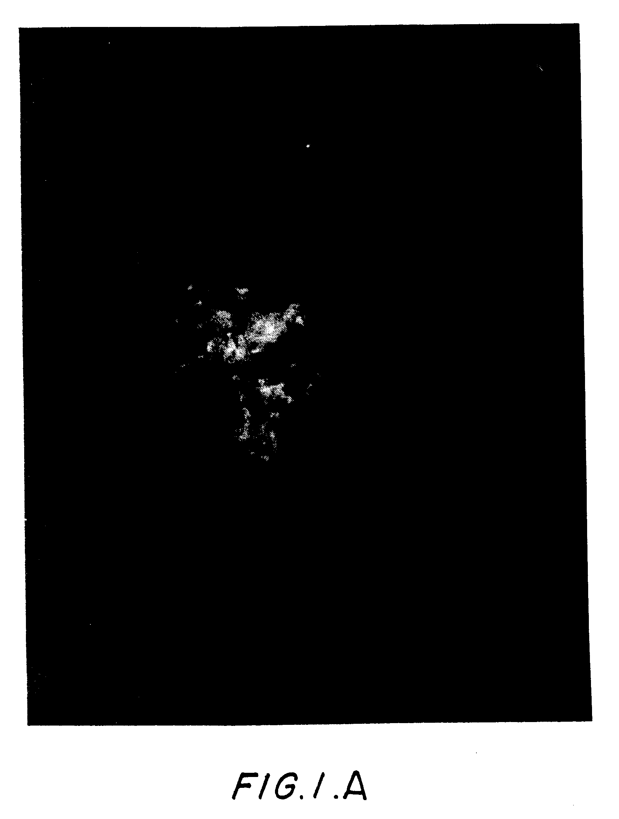

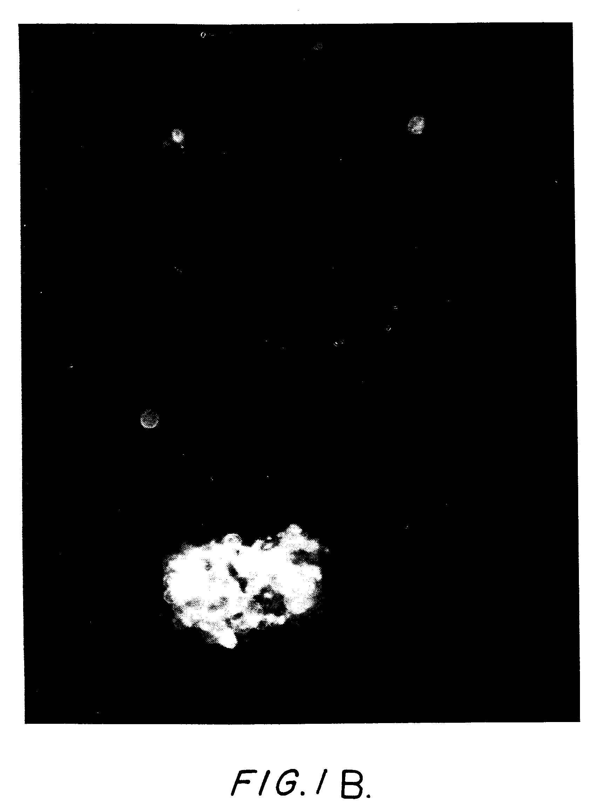

The observation of the fluorescence of the sample and the reference under the fluorescence microscope with blue light excitation shows that the cells of the control sample which has not received any electric pulse have fluorescent green points on the membrane due to the FITC fluorescence. The electroporated sample shows highly fluorescent cells which agglutinate. The agglutination is due to 10F7 Antiglycophorin bonding between cells which have integrated glycophorin. This agglutination gives an idea of the number of glycophorin molecules integrated by cell, which must be of the order of $10^5$ to be able to induce agglutination of cells (see Fig. 1). The results of a pseudo ELISA confirm this order of insertions, and it shows that $4.2 \times 10^5$ glycophorin per cell are inserted (see Example 6).

An explanation for the above is that the presence of $Ca^{++}$ and $Mg^{++}$ ions in low concentration (0.5 mM) in low ionic concentration medium help the glycophorin to be fixed to the plasma membrane of the red blood cell such that, during the pulse, the glycophorin molecules already very close to the membrane will integrate.

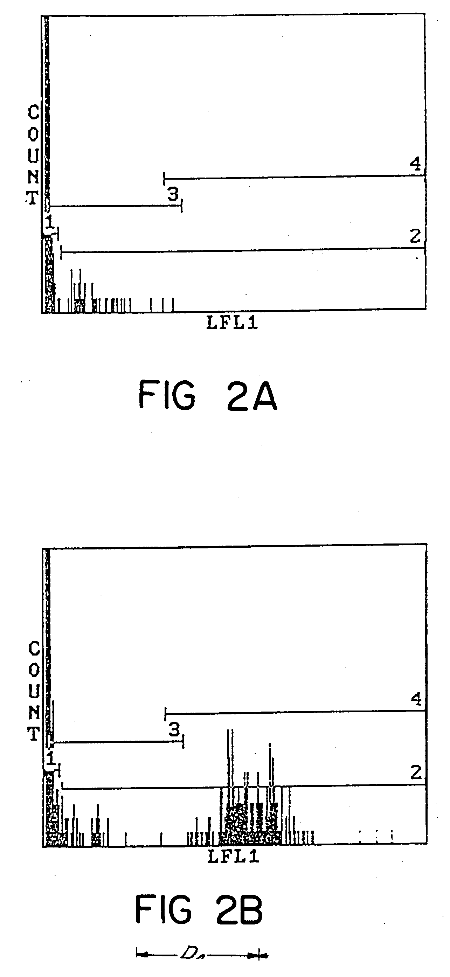

Using cytofluorimetry performed with a Coulter EPICS Profile instrument, it is difficult to ascertain a true estimation of the percentage of protein-integrated cells because of the agglutination of these cells (case of Example 1a). For some single cells, fluorescence can be measured corresponding to a mean value of $2.5 \times 10^5$ fluorescent molecule (FITC) per cell. This number is in agreement with the estimated rate of glycophorin integration necessary to induce agglutination (Fig. 2).

Example 3b:

The inserted CD4 is assayed by different anti-CD4 monoclonal antibodies including OKT4A, OKT4C and OKT4D (Ortho Diagnostic Systems, Inc., Raritan, NJ, U.S.A.), BL4/10T4 (AMAC, Inc., Westbrook, ME, U.S.A.), CYT029 (Cytogen Corp., Princeton, NJ, U.S.A.) and Leu 3A (active epitope with gp120 envelop protein of HIV virus) (Becton Dickinson Immunocytometry System, Mountain View, CA, U.S.A), followed by a phycoerythrin conjugated F(ab')2 fragment goat anti-mouse IgG or fluorescein conjugated affinipure F(ab')2 fragment rabbit anti-mouse IgG.

Control samples are used as references for each corresponding case, where red blood cells subjected to all the steps mentioned in Example 1b, except that glycophorin or CD4 is replaced with bovin serum albumin.

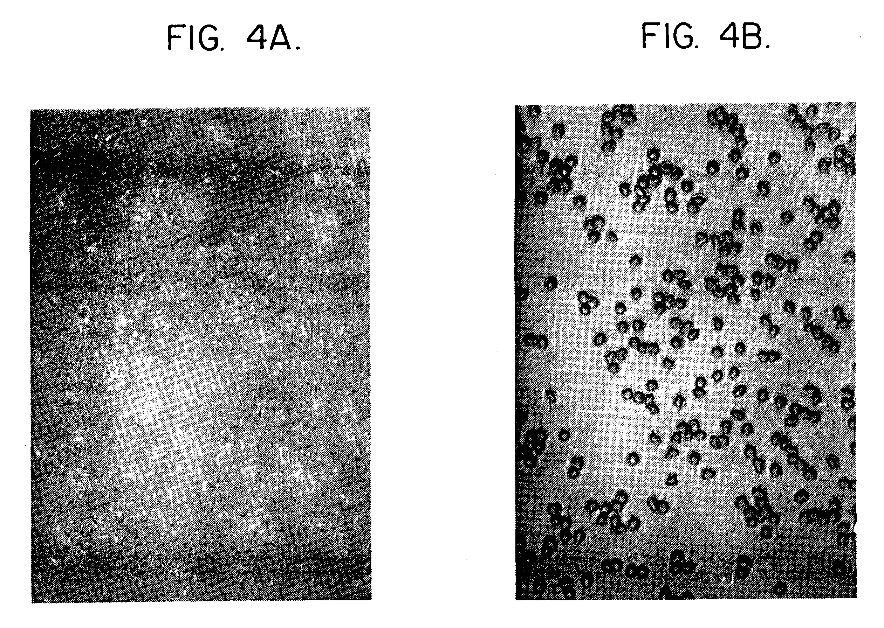

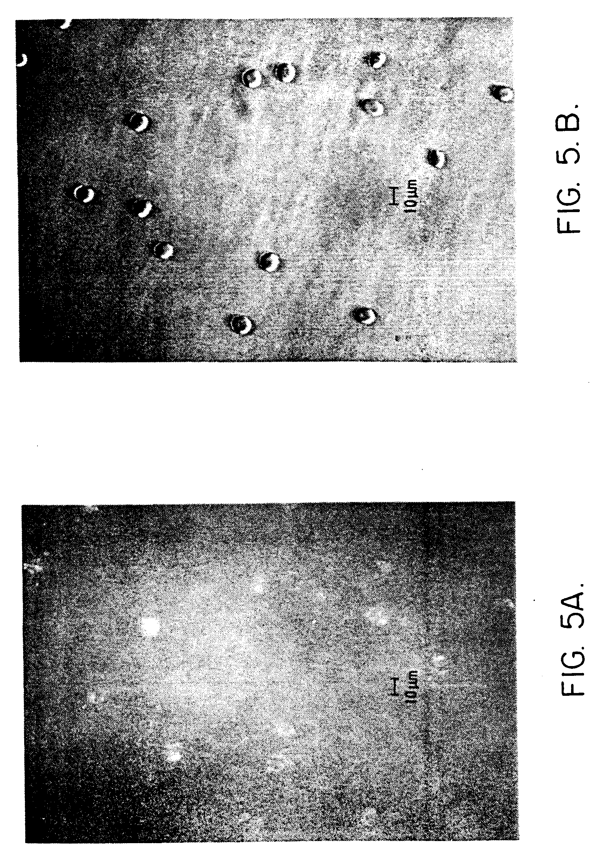

Under the fluorescence microscope, red blood cells bearing the inserted proteins show bright fluorescent points characteristic of the capping of the inserted glycophorin (Fig. 4) into rabbit red blood cells. Fig. 5 shows detection by monoclonal antibodies fluorescently stained of CD4 electroinserted into human red blood cell membranes.

Flow cytometry was performed on a Coulter EPICS Profile instrument. The phycoerythrin was measured with a 575 nm band pass emission filter, while the fluorescein was measured with a 525 nm band pass emission filter. Alignment of the instrument was performed using 5 micrometer diameter Immunocheck beads. The high voltage of the photomultipliers was set up to obtain the characteristic fluorescence peak at the channels of these fluorescent standards, using Immuno-bright beads with different fluorescence intensity. The following histograms were collected for analysis: 1) 90°-sidescatter vs. forward angle scatter; 2) log green or red fluorescence vs. number of cells.

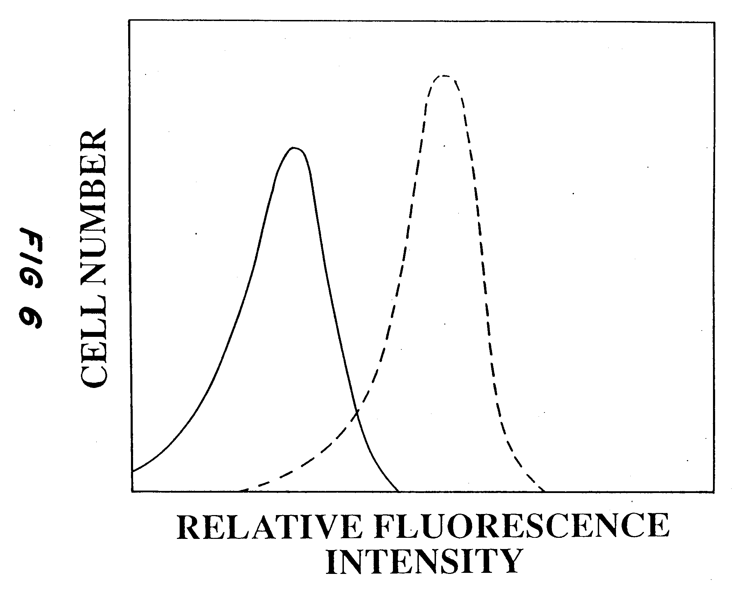

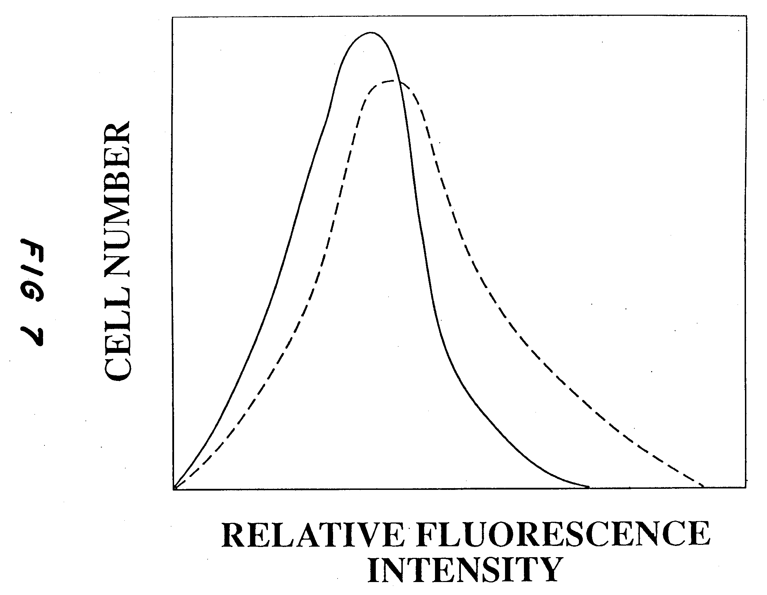

The flow cytometry analysis of the fluorescence intensity and the cell number shows an intensity shift of the whole population of cells with respect to the control sample. This indicates that all the red blood cells have been subjected to the electro-insertion of the xeno-proteins. The mean peak channel of the fluorescence intensity is used to estimate the number of inserted protein molecules per cell. Fig. 6 represents the case of inserted glycophorin into rabbit red blood cells membrane, and Fig. 7 represents the case of inserted CD4 into rabbit red blood cells membrane.

Using standard fluorescent beads (Coulter), the average number of inserted molecules per red blood cell is found to be 2000. The efficiency of insertion (inserted molecules per added molecules per cell) is 1/1000. This efficiency is underestimated because the detection of inserted molecules is mode by immunofluorescence, where active epitopes are detected only, while the added number of molecules is deduced from the weight which does not represent effectively the true number of protein molecules presenting active epitopes.

Example 4: Electro-insertion of Biotinylated Human Glycophorin in Human Red Blood Cells

Another application of the electro-insertion is the insertion of biotinylated human glycophorin in human red blood cells The instruments and the median are the same as described above. The height of the electric field is 1.3 kv/cm, which is below the critical field for electroporation of human red blood cells (2.2 kv/cm).

## Example 5: Biotinylation of Glycophorin

Human glycophorin MN Sigma Chemical Co., St. Louis, Mo., U.S.A.) is dissolved in sodium bicarbonate 0.1M till 0.5 mg/ml. At 4°C and while stirring, an equal volume of 1 mg/ml succinimidyl D-biotin (molecular probes) is added drop by drop in dimethyl-formamide. The solution is incubated overnight at 4°C with stirring. A dialysis four times with KCl 0.15M and one time with 0.3M mannitol give the glycophorin-biotin solution in mannitol used for the electro-insertion.

## Example 6: Pseudo ELISA

In order to get a quantitative evaluation of the integrated molecules of glycophorin per red blood cell, a pseudo ELISA test is used where the glycophorin-biotin molecule is labelled by alkaline phosphatase avidin (Alk. Phos. Avidin) conjugate (ICN). An ELISA plate Costa, Cambridge, Ma., U.S.A.) is used and the color intensity is read by DYNATECH Mr 700 with a 410 nm filter.

First, a standard curve of optical density versus concentration of biotinylated-glycophorin is set, and it is corrected for the background of unbiotinylated glycophorin. A serial dilution of suspended cells bearing glycophorin-biotin is made; after drying at 37°C for 90 minutes, the cells are washed three times with a washing buffer (PBS 7.4, 1% BSA, 0.5% TWEEN 20), after the plates are blocked by adding a given volume of washing buffer and incubating 1 hour at 37°C. After three washes in the washing buffer, an alkaline phosphatase avidine diluted 1:10,000 is added and incubation is conducted for one hour at 37°C. After three washes with the washing buffer and three times with distilled water, the substrate buffer (0.4 mg/ml of para-nitrophenol phosphate in diethanolamine DEA buffer pH 9.8) is added and incubation is conducted for one hour at room temperature. Finally, reaction is stopped with 2.5 N NaOH, and the plates are read at 410 nM.

The counting of the number of cells per well is done by Coulter counter ZM Channelyzer 256.

A psuedo ELISA, similar to that as described above, is conducted to quantify the human glycophorin inserted in mouse red blood cells, but instead of Alk, Phos. Avidin, 10F7 antiglycophorin and Alk. Phos. AP F(ab')$_2$ fragment goat antimouse IgG is employed.

## Example 7: Electro-insertion of Glycophorin-Biotin in Plasma Membrane of Human Red Blood Cells

Red cells from freshly drawn human blood are washed three times 0.145 NaCl in PBS 7.4 buffer and two times in EM. After addition of 0.5 mM of CaCl$_2$ and 0.5 mM of MgCl$_2$, the cells are incubated 5 minutes on ice and 5 minutes at 37°C (hematocrit is 90%). Immediately, a given volume of the solution of glycophorin-biotin is added, such that 1.25 $\mu$g glycophorin per $10^6$ cell are yielded, and then incubation is conducted for five more minutes at 37°C. The suspension is then centrifuged and the supernatent removed to get a hemotocrit of 16%. 25 $\mu$l of the final suspension is electropulsed at 4°C.

Immediately after the pulse, the cells are incubated at 37°C plus one more hour in the presence of RM. Then, the cells are washed one time with the RM (see Example 1a) and two times with 0.145 M NaCl PBS 7.4. Half of the sample is labelled by avidine-FITC (molecular probes) and the other half is used for ELISA.

A control sample which underwent all the steps of this example, except the electric pulse is used as reference.

Moreover, another control has been done to check for the unspecific binding of the avidin-FITC as follows: the RBC have been electroporated exactly in the same manner, but in the absence of glycophorin-biotin and the fluorescence was completely negative.

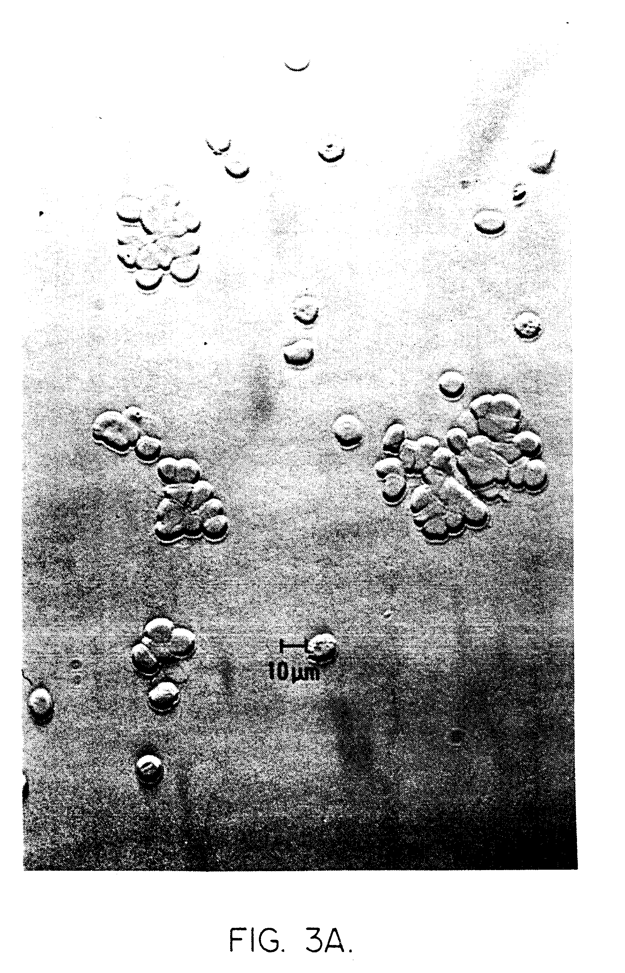

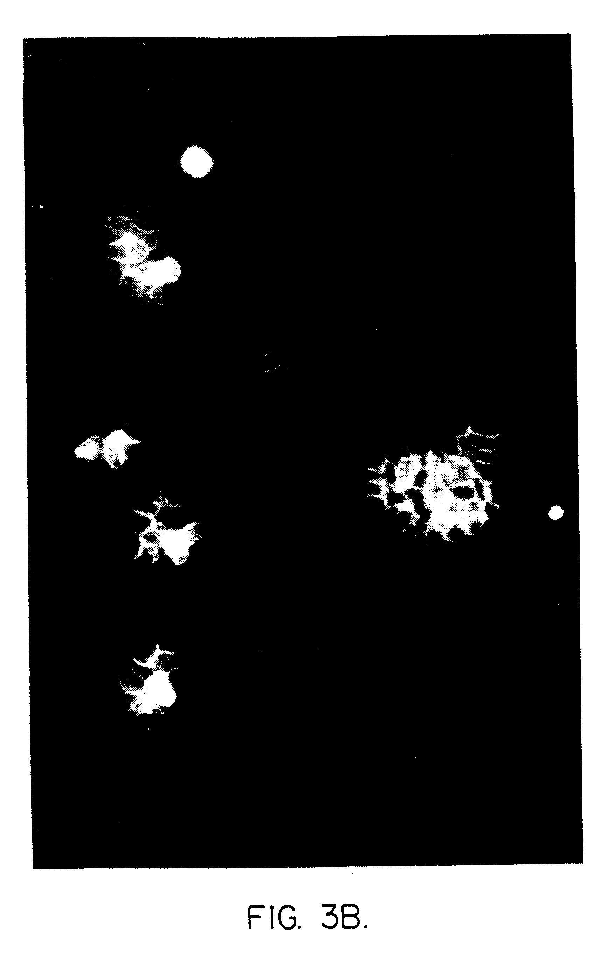

The pseudo-ELISA shows that up to 3.5 x $10^5$ per human red blood cell can be inserted. This order of insertion is in agreement with the agglutination of the red cells bearing biotynilated glycophorin, as shown in Figs. 3a and 3b due to cell-cell bonding by avidin. This common phenomenon of agglutination in this case and in the case of anti-glycoporin antibody is not an artefact but a strong proof of high level of insertion. The control where avidin and anti-glycophorin antibody is added on electropulsed cells does not show any agglutination. This new technique has a powerful application for the insertion of CD4 in red blood cells or the insertion of any other molecule useful for drug targeting.

## Example 8: In Vivo Life Span of $^{125}$I-Glycophorin and $^{125}$I-CD4 Inserted in Rabbit Red Blood Cells

Purified rCD4 and human glycophorin were labeled with $^{125}$I using [$^{125}$I]-Bolton-Hunter Reagent ($^{125}$I-BHR) (New Research Products, Boston, MA, U.S.A.), according to their procedure.

4 ml of blood from a male rabbit (New Zealand white 2.5 to 3kg), obtained by auris arteriopuncture, were collected into a heparinized tube. Proteins were inserted (as explained in Example 1b) in 700 $\mu$l of packed red blood cells. After insertion, red blood cells were labeled by $^{51}$Cr using a sodium chromate Cr51(Na$_2$$^{51}$CRO$_4$) kit from Mallinckrodt (Saint Louis, MO, U.S.A.), according to their procedure. The erythrocyte-CD4$^+$ (glyccphorin$^+$) suspension was reinfused by auris venipuncture into the same rabbit.

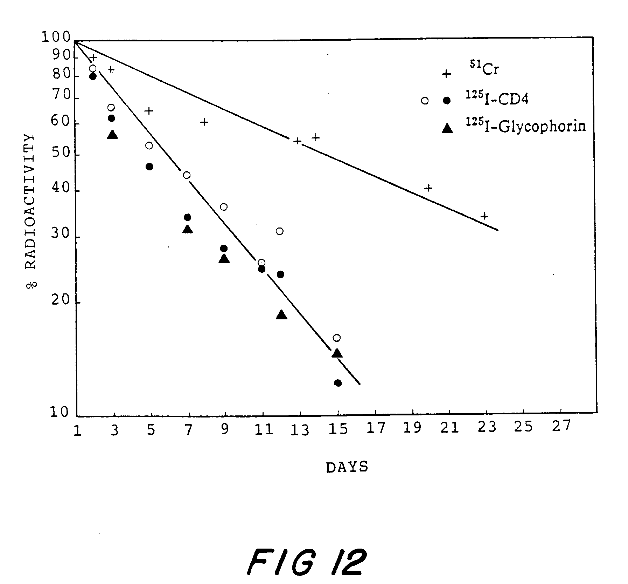

Blood samples (1.5 ml) were measured by an automatic gamma counting system (TM Analytic 1911 Gamma Trac from TM Analytic Inc., Elk Grove Village, IL, U.S.A.). Fig. 12 shows the life span of the red blood cells subjected to electro-insertion and that of the inserted proteins. The chromium analysis shows that electro-insertion does not disturb the life time of the red blood cells in vivo where the half life time is 17 days. The half life time of the inserted proteins is shorter (7 days).

No immune response was detected either against inserted CD4 or glycophorin. Therefore, electro-insertion provides a long lived carrier of full length CD4 which retains its immunological activity in circulation.

CD4$^+$ erythrocytes resuspended in autologous plasma or physiological solution (0.15 M NaCl) at hematocrit 50% can be injected into the blood stream of an AIDS patient for use as a specific scavenger for free HIV virus circulating, circulating gp120 and infected cells presenting the gp120 molecule on their surface. With the latter, RBC (red blood cells)-CD4$^+$ form aggregates which are recognized and phagocyted by the reticulo-endothelial system.

The CD4$^+$ erythrocytes could also be loaded with a specific antiviral drug (e.g., AZT) efficient against HIV injection in order to target it to infected, phagocytic cells.

The electro-insertion process according to the invention could be used to insert other membrane proteins into the erythrocyte membrane in order to treat other diseases involving specific surface antigens or receptors, e.g., hepatitis B virus and malaria, or where targeting drug delivery by means of red blood cells with electro-inserted membrane proteins is desired.

The present invention can also be employed to treat autoimmune diseases by electro-inserting appropriate antibodies in red blood cell membranes and then administering such red blood cell membranes to patients.

Example 9: Electro-Insertion of Glycophorin Into CEM Cell Membrane

The electro-insertion of human glycophorin into human lymphoma T cells (CEM) cell membrane is accomplished using the same procedure decribed above for red blood cells. About 1000 glycophorin molecules per CEM cell have been detected. This result shows that electro-insertion technique can be applied to eukariotic, nucleated cells as well.

Example 10: Spontaneous Insertion Of Glycophorin And CD4 Into Murine, Rabbit and Human Red Blood Cell Membrane

Red cells from freshly drawn blood are washed five times in 0.14 NaCl in PSB 8.8 buffer. Membrane proteins (glycophorin or CD4) lyophilized or suspended in 10 PBS pH 8.8 are added to the red blood cells pellet (hematocrit 90%). After 20 minutes incubation on ice and 3 hours incubation at 37°C, red blood cells are washed one time with plasma and two times with 5 PBS pH 7.4. Depending on the added protein concentration, up to 4500 epitopes are detected by an immunofluorescence assay using flow cytometry analysis.

It will be appreciated that the instant specification and claims are set forth by way of illustration and not limitation, and that various modifications and changes may be made without departing from the spirit and scope of the present invention.

## Claims

1. A method for incorporating a protein into a cell membrane comprising exposing a cell to an electric field while said cell is suspended in an electro-insertion medium in the presence of a buffered solution of the protein to be inserted, said protein bearing a hydrophobic membrane spanning sequence, and then conducting resealing by contacting the resultant cell with a resealing medium.

2. A method according to claim 1, wherein the cell is an animal cell found in the bloodstream.

3. A method according to claim 2, wherein the cell is an erythrocyte.

4. A method according to claim 1, wherein the electric field comprises one or more electric field pulses.

5. A method according to claim 4, wherein the electric field pulses comprise up to eight electric pulses applied sequentially with a time interval of up to 5 seconds between each pulse.

6. A method according to claim 4, wherein the electric field pulse has a height which is the same or below the critical field for electroporation.

**7.** A method according to claim 4, wherein the electric field pulse is at least 400 $\mu$s long.

**8.** A method according to claim 4, wherein the electric field pulse is a square pulse.

**9.** A method according to claim 4, wherein the electric field pulses comprises up to four electric pulses applied sequentially with 15 minute time intervals between each pulse.

**10.** A method according to claim 4, wherein the electric field pulse is up to 1 millisecond.

**11.** A method according to claim 4, wherein the pulse height is 1.3 kv/cm to 2.1 kv/cm.

**12.** A method according to claim 1, wherein the electro-insertion medium comprises an aqueous mixture of mannitol and histidine at a pH of 7.8 or below.

**13.** A method according to claim 1, wherein the resealing occurs in a resealing medium, wherein the resealing medium is an isotonic medium.

**14.** A method according to claim 13, wherein the resealing medium comprises an aqueous mixture of a moiety selected from the group consisting of one or more of KCl, NaCl, BSA, HSA, $MgCl_2$ and glucose, in PBS.

**15.** A method according to claim 1, wherein the suspension has a hematocrit of 0.001% to 98%.

**16.** A method according to claim 1, wherein the protein is a human glycophorin.

**17.** A method according to claim 1, wherein the protein is a CD4 receptor.

**18.** A method according to claim 1, wherein the protein molecules are in a ratio to the cell such that every cell is fully covered by protein before the exposure to the electric field.

**19.** A method according to claim 18, wherein for protein monomers, the ratio is $10^6$ protein molecules per cell.

**20.** A method according to claim 1, wherein the electro-insertion medium is PBS at a pH of up to 8.8.

**21.** A method according to claim 1, wherein the method is conducted at a temperature of 2 °C to 39 °C.

**22.** A method according to claim 1, wherein the method is conducted for a time of 1 to 4 hours.

**23.** A method according to claim 1, wherein the method is conducted at a temperature of 37 °C and the method is conducted for a time of 3 hours.

**24.** A method according to claim 1, wherein the solution further comprises L-alpha-phosphatidic acid dipalmitoyl.

**25.** A method according to claim 1, which further comprises conducting an incubation with a preservative medium after the resealing, said incubation at a temperature of 20 °C to 39 °C for 1 to 2 hours.

**26.** A method according to claim 1, which further comprises conducting an incubation with a preservative medium after the resealing, said incubating at a temperature of 2 to 6 °C for 6 to 12 hours.

**27.** A method according to claim 1, wherein the cell is a nucleated animal cell in culture.

**28.** A method according to claim 27, wherein the cell is CEM.

**29.** Use of the cell bearing the inserted protein being prepared according to the any of the preceeding claims for making a medicine for the treatment of an infectious disease.

**30.** The medicine according to claim 29, wherein the cell is an erythrocyte and the protein molecule is CD4.

**31.** The medicine according to claim 29 for the treatment of AIDS.

**Patentansprüche**

**1.** Ein Verfahren zum Einbau eines Proteins in ein Zellmembran, enthaltend das Exponieren einer Zelle einem elektrischem Feld während die Zelle in einem Medium zum elektrischen Einbau in Gegenwart einer gepufferten Lösung des einzubauenden Proteins suspendiert ist, wobei das Protein eine hydrophobe membranüberspannende Sequenz aufweist und anschließendes Wiederversiegeln durch in Berührung bringen der erhaltenen Zelle mit einem Wiedervesiegelungsmedium.

2. Ein Vorfahren nach Anspruch 1, worin die Zelle eine tierische Zelle des Blutstroms ist.

3. Ein Verfahren nach Anspruch 2, worin die Zelle ein Erythrocyt ist.

4. Ein Verfahren nach Anspruch 1, worin das elektrische Feld einen oder mehrere elektrische Feldimpulse aufweist.

5. Ein Verfahren nach Anspruch 4, worin die elektrischen Feldimpulse bis zu acht elektrische Impulse, die regelmäßig innerhalb eines Zeitintervalls von bis zu 5 Sekunden zwischen jedem Impuls angelegt werden, enthalten.

6. Ein Verfahren nach Anspruch 4, worin der elektrische Feldimpuls eine Stärke gleich oder unterhalb dem kritischen Feld zur Elektroporation aufweist.

7. Ein Verfahren nach Anspruch 4, worin der elektrische Feldimpuls wenigstens 400 $\mu$s lang ist.

8. Ein Verfahren nach Anspruch 4, worin der elektrische Feldimpuls ein Rechteckimpuls ist.

9. Ein Verfahren nach Anspruch 4, worin der elektrische Feldimpuls bis zu vier elektrische Impulse umfaßt, die regelmäßig innerhalb von Zeitintervellen von 15 Minuten zwischen jedem Impuls angelegt werden.

10. Ein Verfahren nach Anspruch 4, worin der elektrische Feldimpuls eine Millisekunde dauert.

11. Ein Verfahren nach Anspruch 4, worin die Pulsstärke 1,3 kv/cm bis 2,1 kv/cm beträgt.

12. Ein Verfahren noch Anspruch 1, worin das Medium zum elektrischen Einbau ein wäßriges Gemisch von Mannit und Histidin bei einem pH-Wert von 7,8 oder darunter enthält.

13. Ein Verfahren nach Anspruch 1, worin das Versiegeln in einem Versiegelungsmedium erfolgt, wobei das Versiegelungsmedium ein isotonisches Medium ist.

14. Ein Verfahren nach Anspruch 13, worin das Versiegelungsmedium ein wäßriges Gemisch eines Anteils, ausgewählt aus der aus einem oder mehreren von KCl, NaCl, RSA, HSA, MgCl₂ und Glucose in PBS bestehenden Gruppe ist.

15. Ein Verfahren nach Anspruch 1, worin die Suspension einen Hämatokrit von 0,001 bis 98% besitzt.

16. Ein Verfahren nach Anspruch 1, worin das Protein ein menschliches Glycophorin ist.

17. Ein Verfahren nach Anspruch 1, worin das Protein ein CD4-Rezeptor ist.

18. Ein Verfahren nach Anspruch 1, worin die Proteinmoleküle in einem Verhältnis zu der Zelle derart vorliegen, daß jede Zelle vollständig durch Protein bedeckt ist, bevor sie einem elektrischem Feld ausgesetzt wird.

19. Ein Vorfahren nach Anspruch 18, worin die Proteinmonomere in einem Verhältnis von $10^6$ Proteinmolekülen je Zelle eingesetzt werden.

20. Ein Verfahren nach Anspruch 1, worin das Medium zum elektrischan Einbau PBS mit einem pH-Wert von bis zu 8,8 ist.

21. Ein Verfahren nach Anspruch 1, das bei einer Temperatur von 2 bis 39 °C durchgeführt wird.

22. Ein Verfahren nach Anspruch 1, das über eine Zeit von 1 bis 4 Stunden durchgeführt wird.

23. Ein Verfahren nach Anspruch 1, welches bei einer Temperatur von 37 °C 3 Stunden lang durchgeführt wird.

24. Ein Verfahren nach Anspruch 1, worin die Lösung ferner L-alpha-Dipalmitoylphosphatidsäure enthält.

25. Ein Verfahren nach Anspruch 1, welches ferner die Durchführung der Inkubation mit einem Konservierungsmittelmedium nach dem Versiegeln umfaßt, wobei die Inkubation bei einer Temperatur von 20 bis 39 °C 1 bis 2 Stunden lang durchgeführt wird.

26. Ein Verfahren nach Anspruch 1, welches ferner die Inkubation mit einem Konservierungsmittelmedium nach dem Versiegeln umfaßt, wobei die Inkubation bei einer Temperatur von 2 bis 6 °C 6 bis 12 Stunden lang durchgeführt wir

27. Ein Verfahren nach Anspruch 1, worin die Zelle eine tierische, kernhaltige Zelle in Kultur ist

28. Verfahren nach Anspruch 27, worin die Zelle CEM ist.

**29.** Verwendung einer Zelle, die ein eingefügtes Protein trägt und nach einem der vorangegangenen Ansprüche hergestellt worden, ist zur Herstellung eines Arzneimittels gegen eine infektiöse Krankheit.

**30.** Des Arzneimittel nach Anspruch 29, worin die Zelle ein Erythrocyt und das Proteinmolekül CD4 ist.

**31.** Das Arzneimittel nach Anspruch 29 zur Behandlung von AIDS.

**Revendications**

**1.** Procédé pour l'incorporation d'une protéine dans une membrane cellulaire comprenant les étapes consistant à exposer une cellule à un champ électrique, ladite cellule étant mise en suspension dans un milieu d'électro-insertion en présence d'une solution tamponnée de la protéine devant être insérée, ladite protéine portant une séquence de recouvrement de la membrane hydrophobe et puis réaliser la resoudre par mise en contact de la cellule résultante avec un milieu de resoudre.

**2.** Procédé selon la revendication 1, dans lequel la cellule est une cellule animale présente dans le flux sanguin.

**3.** Procédé selon la revendication 2, dans lequel la cellule est un érythrocyte.

**4.** Procédé selon la revendication 1, dans lequel le champ électrique comprend une ou plusieurs impulsions de champs électriques.

**5.** Procédé selon la revendication 4, dans lequel les impulsions de champs électriques comprennent jusqu'à huit impulsions électriques appliquées à intervalles réguliers, l'intervalle entre deux impulsions étant de 5 secondes.

**6.** Procédé selon la revendication 4, dans lequel l'impulsion de champ électrique a une hauteur égale ou inférieure à celle du champ critique permettant l'électroporation.

**7.** Procédé selon la revendication 4, dans lequel l'impulsion de champ électrique dure au moins 400 $\mu$s.

**8.** Procédé selon la revendication 4, dans lequel l'impulsion de champ électrique est une impulsion carrée.

**9.** Procédé selon la revendication 4, dans lequel les impulsions de champs électriques comprennent jusqu'à quatre impulsions électriques appliquées à intervalles réguliers, l'intervalle entre deux impulsions étant de 15 minutes.

**10.** Procédé selon la revendication 4, dans lequel l'impulsion de champ électrique dure jusqu'à 1 milliseconde.

**11.** Procédé selon la revendication 4, dans lequel la hauteur d'impulsion est de 1,3 kV/cm à 2,1 kV/cm.

**12.** Procédé selon la revendication 1, dans lequel le milieu d'électro-insertion comprend un mélange aqueux de mannitol et d'histidine à un pH de 7,8 ou inférieur.

**13.** Procédé selon la revendication 1, dans lequel la resoudre a lieu dans un milieu de resoudre dans lequel le milieu de resoudre est un milieu isotonique.

**14.** Procédé selon la revendication 13, dans lequel le milieu de resoudre comprend un mélange aqueux d'un ou plusieurs composés choisis parmi le groupe constitué de KCl, NaCl, BSA, HSA, MgCl$_2$ et glucose dans le PBS.

**15.** Procédé selon la revendication 1, dans lequel la suspension a un hématocrite de 0,001% à 98%.

**16.** Procédé selon la revendication 1, dans lequel la protéine est une glycophorine humaine.

**17.** Procédé selon la revendication 1, dans lequel la protéine est un récepteur de CD4

**18.** Procédé selon la revendication 1, dans lequel le rapport des molécules de protéines aux cellules est tel que chaque cellule est complètement recouverte par une protéine avant l'exposition au champ électrique.

**19.** Procédé selon la revendication 18, dans lequel, pour des monomères de protéines, le rapport est de 10$^6$ molécules de protéine par cellule.

**20.** Procédé salon la revendication 1, dans lequel le milieu d'électro-insertion est du PBS à un pH pouvant atteindre jusqu'à 8,8.

**21.** Procédé selon la revendication 1, dans lequel le procédé est réalisé à une température de 2 à 39°C.

22. Procédé selon la revendication 1, dans lequel le procédé est réalisé pendant une durée de 1 à 4 heures.

23. Procédé selon la revendication 1, dans lequel la procédé est réalisé à une température de 37°C et le procédé est réalisé pendant un temps égal à 3 heures.

24. Procédé selon la revendication 1, dans lequel le solution contient en outre de l'acide L-$\alpha$-phosphatidique de dipalmitoyle.

25. Procédé selon la revendication 1, comprenant en outre, après l'étape de resoudure, une étape d'incubation dans un milieu préservateur, ladite incubation ayant lieu à une température de 20 à 39°C pendant 1 à 2 heures.

26. Procédé selon la revendication 1, comprenant en outre après l'étape de resoudure, une étape d'incubation dans un milieu préservateur, ladite incubotion ayant lieu à une température de 2 à 6°C pendant 6 à 12 heures.

27. Procédé selon la revendication 1, dans lequel la cellule est une cellule animale nuclée en culture.

28. Procédé selon la revendication 27, dans lequel la cellule est le CEM.

29. Utilisation d'une cellule portant une protéine insérée, préparée selon l'une quelconque des revendications précédentes pour la préparation d'un médicament destiné au traitement de maladies infectieuses.

30. Médicament selon la revendication 29, dans lequel la cellule est un érythrocyte et la molécule de protéine est le CD4.

31. Médicament selon la revendication 29, pour le traitement du SIDA.

FIG.I.A

FIG. I B.

FIG 2A

FIG 2B

FIG. 3A.

FIG. 3B.

FIG. 4B.

FIG. 4A.

FIG. 5A.

FIG. 5. B.

EP 0 362 758 B1

FIG 6

FIG 7

FIG.8

FIG 9

FIG 10

FIG.II

EP 0 362 758 B1

FIG 12